# NEUE EUROPÄISCHE PATENTSCHRIFT

(11) **EP 1 015 410 B2**
(45) Veröffentlichungstag und Bekanntmachung des Hinweises auf die Entscheidung über den Einspruch: **26.09.2007**
(45) Hinweis auf die Patenterteilung: 03.04.2002
(21) Anmeldenummer: 98948948.9
(22) Anmeldetag: 09.09.1998
(51) Int. Cl.: C07C 51/43

(54) **VERFAHREN ZUR HERSTELLUNG VON ACRYLSÄURE UND METHACRYLSÄURE**
METHOD FOR PRODUCING ACRYLIC ACID AND METHACRYLIC ACID
PROCEDE POUR LA PREPARATION D'ACIDE ACRYLIQUE ET D'ACIDE METHACRYLIQUE

(30) Priorität: 12.09.1997 DE 19740252
(43) Veröffentlichungstag der Anmeldung: 05.07.2000
(73) Patentinhaber: BASF Aktiengesellschaft, 67056 Ludwigshafen (DE)
(72) Erfinder: ECK, Bernd, D-68519 Viernheim (DE); MACHHAMMER, Otto, D-68163 Mannheim (DE); PROLL, Theo, D-67098 Bad Dürkheim (DE); SCHLIEPHAKE, Volker, D-67105 Schifferstadt (DE); THIEL, Joachim, D-67433 Neustadt (DE); BRÖLLOS, Klaus, D-64342 Seeheim-Jugenheim (DE)
(74) Vertreter: Isenbruck, Günter
(86) Internationale Anmeldenummer: PCT/EP1998/005753
(87) Internationale Veröffentlichungsnummer: WO 1999/014181

(56) Entgegenhaltungen:
- EP-A- 0 675 100
- EP-A- 0 784 046
- EP-A- 0 792 867
- WO-A-98/01415
- DE-A- 19 606 877
- US-A- 4 780 568

## Beschreibung

Die vorliegende Erfindung betrifft ein Verfahren zur Herstellung von Acrylsäure oder Methacrylsäure.

Acrylsäure ist eine bedeutende Grundchemikalie. Aufgrund ihrer sehr reaktionsfähigen Doppelbindung sowie der Säurefunktion eignet sie sich insbesondere als Monomeres zur Herstellung von Polymerisaten. Von der hergestellten Menge an Acrylsäuremonomeren wird der größere Teil vor der Polymerisation - zu z.B. Klebstoffen, Dispersionen oder Lacken - verestert. Nur der kleinere Teil der hergestellten Acrylsäuremonomeren wird direkt - zu z.B. "Superabsorbern" - polymerisiert. Während im allgemeinen bei der direkten Polymerisation der Acrylsäure Monomere hoher Reinheit benötigt werden, sind die Anforderungen an die Reinheit der Acrylsäure nicht so hoch, wenn diese vor der Polymerisation verestert wird.

Es ist allgemein bekannt, daß Acrylsäure durch heterogen katalysierte Gasphasenoxidation von Propen mit molekularem Sauerstoff an im festen Aggregatzustand befindlichen Katalysatoren bei Temperaturen zwischen 200 und 400 °C einstufig oder zweistufig über Acrolein hergestellt werden kann (vgl. z.B. DE-A-1 962 431, DE-A-2 943 707, DE-C-1 205 502, DE-A-195 08 558, EP-A-0 257 565, EP-A-0 253 409, DE-A-2 251 364, EP-A-0 117 146, GB-B-1 450 986 und EP-A-0 293 224). Hierbei werden oxidische Mehrkomponenten-Katalysatoren z.B. auf der Basis von Oxiden der Elemente Molybdän, Bismut und Eisen (in der 1. Stufe) bzw. Molybdän und Vanadium (in der 2. Stufe) eingesetzt.

Aus DE-C-2 136 396 ist bekannt, die Acrylsäure aus den bei der katalytischen Oxidation von Propen bzw. Acrolein erhaltenen Reaktionsgasen durch Gegenstromabsorption mit einem Gemisch aus etwa 75 Gew.-% Diphenylether und etwa 25 Gew.-% Diphenyl abzutrennen. Weiterhin ist aus DE-A-2 449 780 das Abkühlen des heißen Reaktionsgases durch Teilverdampfen des Lösungsmittels in einem Direktkondensator (Quenchapparat) vor der Gegenstromabsorption bekannt. Problematisch ist hierbei sowie bei weiteren Verfahrensschritten der Anfall von Feststoffen in den Apparaten, der die Anlagenverfügbarkeit reduziert. Gemäß DE-A-4 308 087 kann dieser Feststoffanfall dadurch reduziert werden, indem man dem relativ unpolaren Lösungsmittelgemisch aus Diphenylether und Diphenyl ein polares Lösungsmittel wie Dimethylphthalat in einer Menge von 0,1 bis 25 Gew.-% zufügt.

Neben der oben beschriebenen Absorption des die Acrylsäure enthaltenden Reaktionsprodukts in ein hochsiedendes Lösungsmittelgemisch sehen andere bekannte Verfahren eine Totalkondensation von Acrylsäure und des weiterhin bei der katalytischen Oxidation entstehenden Reaktionswassers vor. Dabei entsteht eine wäßrige Acrylsäurelösung, die über Destillation mit einem azeotropen Mittel (vgl. DE-C-3 429 391, JP-A-1 124 766, JP-A-7 118 766, JP-A-7 118 966-R, JP-A-7 118 968-R, JP-A-7 241 885) oder über ein Extraktionsverfahren (vgl. DE-A-2 164 767, JP-A-5 81 40-039 und JP-A-4 80 91 013) weiter aufgearbeitet werden kann. In EP-A-0 551 111 wird das mittels katalytischer Gasphasenoxidation hergestellte Gemisch von Acrylsäure und Nebenprodukten mit Wasser in einem Absorptionsturm in Berührung gebracht und die erhaltene wäßrige Lösung in Anwesenheit eines Lösungsmittels, das mit polaren Leichtsiedern wie Wasser oder Essigsäure ein Azeotrop bildet, destilliert. DE-C-2 323 328 beschreibt die Abtrennung von Acrylsäure aus einer wäßrigen Butanol-Acrylsäure-Veresterungsablauge durch Extraktion mit einem speziellen Gemisch organischer Lösungsmittel.

Nachteilig bei den oben beschriebenen Verfahren ist, daß zur Extraktion oder Absorption ein organisches Lösungsmittel verwendet wird, das in einer weiteren Verfahrensstufe wie eine Rektifikation bei hoher thermischer Belastung wieder abgetrennt wird. Hierbei besteht die Gefahr einer Polymerisierung der Acrylsäure.

JP-A-07 082 210 beschreibt ein Verfahren zur Reinigung von Acrylsäure, die neben Acrylsäure Essigsäure, Propionsäure, Acrolein und Furfural enthält. Bei diesem Verfahren wird nach Zugabe von Wasser eine Kristallisation im Vakuum durchgeführt, wobei nach Abtrennung und Waschen der Acrylsäurekristalle eine Reinheit von 99,6% erreicht wird. Das japanische Patent 45-32417 offenbart ein Verfahren, bei dem eine wäßrige Acrylsäurelösung bzw. Methacrylsäurelösung, die zusätzlich Essigsäure und Propionsäure enthält, mit Heptan oder Toluol extrahiert wird und anschließend Wasser durch Destillation aus dem Extrakt entfernt wird. In der nächsten Stufe wird der verbleibende Extrakt auf -20 bis -80 °C abgekühlt, um eine Kristallisation von Acrylsäure bzw. Methacrylsäure herbeizuführen. Die Kristalle werden abgetrennt, und die Mutterlauge wird dem Extraktionsprozeß rückgeführt. Gemäß dieser Patentschrift ist die Verwendung eines organischen Lösungs- bzw. Extraktionsmittels notwendig, da ansonsten die Lösung, wenn sie abgekühlt wird, sich verfestigt, ohne daß Kristalle ausfallen. Nachteilig bei diesem Verfahren ist neben dem Zusatz eines organischen Lösungsmittels, daß zur Abtrennung von Wasser eine Destillation durchgeführt werden muß. Das kanadische Patent 790 625 betrifft einen weiteren Reinigungsprozeß für Rohacrylsäure durch fraktionierte Kristallisation. Dabei wird im Falle von Propionsäure als Hauptverunreinigung der Rohacrylsäure die Temperatur nicht unter die peritektische Temperatur des Systems Acrylsäure-Propionsäure abgesenkt, während im Falle von Essigsäure als Hauptverunreinigung die Temperatur nicht unter die eutektische Temperatur des Systems Acrylsäure-Essigsäure abgesenkt wird. Die zur Kristallisation eingesetzte Acrylsäure wird hierbei nach herkömmlichen Verfahren hergestellt, zum Beispiel durch Gasphasenoxidation von Propen oder Acrolein, und anschließend einer Vorreinigung durch herkömmliche bekannte Verfahren, z.B. Extraktion, unterworfen. Gemäß den Angaben der Patentschrift wird die Kristallisation der Acrylsäure vorzugsweise im wesentlichen in Abwesenheit von Wasser durchgeführt.

In EP-A-0 616 998 wird ein Verfahren zur Reinigung von Acrylsäure mittels einer Kombination von dynamischer und statischer Kristallisation beschrieben, wobei als Einsatzprodukt vorgereinigte Acrylsäure, zum Beispiel destillativ vorgereinigte Acrylsäure, verwendet wird.

Den in den obigen Dokumenten beschriebenen Verfahren ist gemeinsam, daß sie eine Vorreinigung der Acrylsäure vor der Kristallisation erfordern. Da bei der Vorreinigung in der Regel organische Lösungsmittel eingesetzt werden, die anschließend bei hoher thermischer Belastung wieder abgetrennt werden, besteht hierbei immer das Problem einer unerwünschten Polymerisation der Acrylsäure.

Aus EP-A-0 002 612, das ein Verfahren zur Reinigung von in wäßriger Lösung vorliegender Acrylsäure durch fraktionierte Kristallisation betrifft, ist der Zusatz von Salzen zur Acrylsäurelösung bekannt, um das Eutektikum Wasser-Acrylsäure aufzubrechen, das bei 63% Volumengehalt Acrylsäure liegt.

EP-A-0 675 100 beschreibt ein Verfahren zur Herstellung α,β-ungesättigter C₃-C₆-Carbonsäuren, z.B. Methacrylsäure, durch oxidative Dehydrierung der entsprechenden gesättigten C₃-C₆-Carbonsäure gefolgt von Schmelzkristallisation mit anschließender fraktionierter Destillation oder gefolgt von fraktionierter Destillation mit anschließender Schmelzkristallisation.

Die Aufgabe der vorliegenden Erfindung bestand darin, ein Verfahren zu schaffen, bei dem Acrylsäure oder Methacrylsäure ohne aufwendige Verfahrensstufen in hoher Reinheit erhalten wird.

Die Lösung dieser Aufgabe geht aus von einem Verfahren zur Abtrennung von Acrylsäure oder Methacrylsäure aus einem gasförmigen Gemisch, das als Rohprodukt der katalytischen Gasphasenoxidation von C₃-/C₄-Alkanen, -Alkenen, -Alkanolen und/oder-Alkanolen und/oder Vorstufen davon vorliegt.

Das erfindungsgemäße Verfahren zur Abtrennung von Acrylsäure oder Methacrylsäure ist dadurch gekennzeichnet, daß
a) das gasförmige Gemisch kondensiert wird,
b) Acrylsäure oder Methacrylsäure aus der in Stufe a) erhaltenen Lösung kristallisiert wird,
c) die erhaltenen Kristalle aus der Mutterlauge der Stufe b) abgetrennt und
d) mindestens eine Teilmenge der Mutterlauge aus Stufe c) nach der Abtrennung in die Stufe a) zurückgeführt wird.

Es wurde gefunden, daß Acrylsäure oder Methacrylsäure aus einem gasförmigen Produktgemisch, das einer Kondensation unterzogen wird, direkt aus der bei der Kondensation entstehenden Lösung auskristallisiert werden kann. Besonders wichtig ist dabei, daß es hierzu keiner weiteren Reinigungsstufe und keines Zusatzes von Hilfsstoffen bedarf.

In einer bevorzugten Ausführungsform wird die Kondensation in Stufe (a) in einer Kolonne durchgeführt. Weitere bevorzugte Ausführungsformen der Erfindung ergeben sich aus der nachfolgenden Beschreibung und dem Beispiel.

Bei dem erfindungsgemäßen Verfahren wird die Acrylsäure oder Methacrylsäure direkt und unmittelbar ohne weitere Zwischen- oder Reinigungsstufen und ohne Zusatz von Hilfsstoffen aus der Lösung auskristallisiert, die bei der Kondensation des Produktgemischs entsteht. Dieses Produktgemisch hat die Zusammensetzung eines bei der katalytischen Gasphasenoxidation zu der Säure entstehenden Reaktionsprodukts.

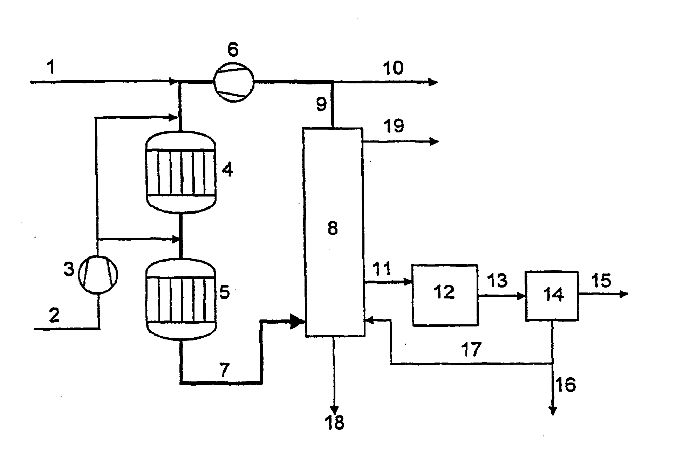

Die einzige Figur zeigt ein bevorzugtes Ausführungsbeispiel zur Durchführung des erfindungsgemäßen Verfahrens.

### Herstellung eines gasförmigen Produktgemisches enthaltend Acrylsäure oder Methacrylsäure

Es wird zunächst ein gasförmiges Produktgemisch hergestellt, das die Zusammensetzung eines Reaktionsgemischs der katalytischen Gasphasenoxidation von C₃- bzw. C₄-Alkanen, -Alkenen, -Alkanolen und/- oder -Alkanalen und/oder Vorstufen davon zu Acrylsäure oder Methacrylsäure hat. Besonders vorteilhaft wird das gasförmige Produktgemisch durch katalytische Gasphasenoxidation von Propen, Propan, Acrolein, tert.-Butanol, Isobuten, Isobutan, Isobutyraldehyd, Methacrolein, Isobuttersäure oder Methyl-tert.-butylether hergestellt. Als Ausgangsverbindungen können alle Vorstufen der oben genannten C₃-/C₄-Verbindungen verwendet werden, bei denen sich die eigentliche C₃-/C₄-Ausgangsverbindung erst intermediär während der Gasphasenoxidation bildet. Beispielhaft genannt für die Herstellung der Methacrylsäure seien Methyl-tert.-butylether oder Isobuttersäure. Sowohl Acrylsäure als auch Methacrylsäure können direkt aus Propan bzw. Isobutan hergestellt werden.

Als Rohprodukt liegt ein gasförmiges Gemisch der katalytischen Gasphasenoxidation von C₃-/C₄-Alkanen, -Alkenen, -Alkanolen und/oder -Alkanalen und/oder Vorstufen davon zu Acrylsäure oder Methacrylsäure vor.

Besonders vorteilhaft ist die katalytische Gasphasenreaktion von Propen und/oder Acrolein zu Acrylsäure mit molekularem Sauerstoff nach bekannten Verfahren, insbesondere wie sie in den oben genannten Druckschriften beschrieben sind. Vorzugsweise wird hierbei bei Temperaturen zwischen 200 und 450 °C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der ersten Stufe (Oxidation von Propen zu Acrolein) und der Oxide von Molybdän und Vanadium in der zweiten Stufe (Oxidation von Acrolein zu Acrylsäure) eingesetzt. Diese Umsetzungen werden beispielsweise einstufig oder zweistufig durchgeführt. Wird Propan als Ausgangsstoff verwendet, so kann dieses zu einem Propen-/Propan-Gemisch umgesetzt werden durch: katalytische Oxidehydrierung, wie z. B. in Catalysis Today 24 (1995), 307 - 313 oder US-A-5 510 558 beschrieben; durch homogene Oxidehydrierung, wie z. B. in CN-A-1 105 352 beschrieben; oder durch katalytische Dehydrierung, wie z. B. in EP-A-0 253 409, DE-A-195 08 558, EP-A-0 293 224 oder EP-A-0 117 146 beschrieben. Geeignete Propen-/Propan-Gemische sind auch Raffineriepropen (70 % Propen und 30 % Propan) oder Crackerpropen (95 % Propen und 5 % Propan). Grundsätzlich können Propen-/Propan-Gemische wie die o. g. mit Sauerstoff oder Luft oder einem Gemisch aus Sauerstoff und Stickstoff jeder Zusammensetzung zu Acrolein und Acrylsäure oxidiert werden. Bei Einsatz eines Propen-/Propan-Gemischs wirkt Propan als Verdünnungsgas und/oder Reaktant. Ein geeignetes Verfahren ist auch in EP-B-0 608 838 beschrieben, bei dem Propan als Reaktant direkt zu Acrylsäure umgesetzt wird.

Die Umsetzung von Propen zu Acrylsäure ist stark exotherm. Das Reaktionsgas, das neben den Edukten und Produkten vorteilhafterweise ein inertes Verdünnungsgas, z.B. Kreisgas (siehe unten), Luftstickstoff, einem oder mehrere gesättigte C₁-C₆-Kohlenwasserstoffe, insbesondere Methan und/oder Propan, und/oder Wasserdampf enthält, kann daher nur einen kleinen Teil der Reaktionswärme aufnehmen. Obwohl die Art der verwendeten Reaktoren an sich keiner Beschränkung unterliegt, werden meist Rohrbündelwärmetauscher verwendet, die mit dem Oxidationskatalysator gefüllt sind, da bei diesen der überwiegende Teil der bei der Reaktion freiwerdenden Wärme durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure Formaldehyd, weitere Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 bis 98 Gew.-%, inerte Verdünnungsgase. Als inerte Verdünnungsgase sind insbesondere gesättigte C₁-C₆-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten.

Die Methacrylsäure kann analog zu Acrylsäure durch katalytische Gasphasenreaktion von C₄-Ausgangsverbindungen mit molekularem Sauerstoff hergestellt werden. Besonders vorteilhaft ist die Methacrylsäure, z. B. durch katalytische Gasphasenoxidation von Isobuten, Isobutan, tert.-Butanol, iso-Butyraldehyd, Methacrolein oder Methyl-tert.-butylether erhältlich. Als Katalysatoren werden ebenfalls übergangsmetallische Mischoxidkatalysatoren (z. B. Mo, V, W und/oder Fe) verwendet, wobei die Umsetzung beispielsweise einstufig oder mehrstufig durchgeführt wird. Besonders geeignete Verfahren sind solche, bei denen die Herstellung ausgehend von Methacrolein erfolgt, insbesondere dann, wenn das Methacrolein durch gasphasenkatalytische Oxidation von tert.-Butanol, Isobutan oder Isobuten oder durch Umsetzung von Formaldehyd mit Propionaldehyd gemäß EP-B-0 092 097 oder EP-B-0 058 927 erzeugt wird. Somit besteht auch die Möglichkeit, Methacrylsäure zweistufig herzustellen durch (I) Kondensation von Propionaldehyd mit Formaldehyd (in Gegenwart eines sekundären Amins als Katalysator) zu Methacrolein und (II) anschließende Oxidation des Methacroleins zu Methacrylsäure. Ein weiteres geeignetes Verfahren ist in EP-B-0 608 838 beschrieben, bei dem Isobutan als Reaktant direkt zu Methacrylsäure umgesetzt werden kann.

Ebenso wie bei der Herstellung der Acrylsäure wird nicht reine Methacrylsäure, sondern ein gasförmiges Gemisch erhalten, das neben der Methacrylsäure als Nebenkomponenten im wesentlichen nicht umgesetztes Methacrolein und/oder Wasserdampf, Kohlenmonoxid, Kohlendioxid, Stickstoff, Sauerstoff, Essigsäure, Propionsäure, weitere Aldehyde und Maleinsäureanhydrid enthalten kann. Das erfindungsgemäße Verfahren wird insbesondere dann eingesetzt, wenn das Reaktionsgemisch, 0,02 bis 2 Gew.-% Methacrolein bezogen auf das gesamte Reaktionsgemisch und ansonsten im wesentlichen die gleichen entsprechenden Bestandteile wie bei der Herstellung der Acrylsäure enthält.

### Stufe (a):

In Stufe (a) wird das, Acrylsäure oder Methacrylsäure enthaltende gasförmige Produktgemisch einer Kondensation, insbesondere einer Partial- oder Totalkondensation unterzogen, wobei eine Lösung erhalten wird.

Die Kondensation wird vorzugsweise in einer Kolonne durchgeführt. Hierbei wird eine Kolonne mit trennwirksamen Einbauten, insbesondere mit Packungen, Füllkörpern und/oder Böden, vorzugsweise Glockenböden, Siebböden, Ventilböden und/oder Dual-Flow-Böden, eingesetzt. Dabei werden die kondensierbaren Komponenten des hergestellten gasförmigen Produktgemisches fraktioniert durch Kühlung auskondensiert. Da das Gasgemisch infolge der Verunreinigungen und Verdünnungsgase eine Schwersieder-, Mittelsieder- und Leichtsiederfraktion sowie nichtkondensierbare Komponenten enthält, können bei der Kolonne an den entsprechenden Stellen ein oder mehrere Seitenabzüge vorgesehen sein. Im Gegensatz zu einer üblichen Kondensation ermöglicht eine Kondensation in einer Kolonne somit bereits eine Auftrennung in die einzelnen Komponenten. Geeignete Kolonnen umfassen wenigstens eine Kühlvorrichtung, wofür sich alle gängigen Wärmeübertrager oder Wärmetauscher, bei denen die bei der Kondensation gebildete Wärme indirekt (extern) abgeführt wird, eignen. Bevorzugt sind Rohrbündelwärmetauscher, Plattenwärmetauscher und Luftkühler. Geeignete Kühlmedien sind Luft bei dem entsprechenden Luftkühler und Kühlflüssigkeiten, insbesondere Wasser, bei anderen Kühlvorrichtungen. Ist nur eine Kühlvorrichtung vorgesehen, so wird diese am Kopf der Kolonne eingebaut, in dem die Leichtsiederfraktion auskondensiert wird. Da das Acrylsäure-bzw. Methacrylsäure-haltige Gasgemisch mehrere Fraktionen enthält, ist es zweckmäßig, mehrere Kühlvorrichtungen in verschiedenen Abschnitten der Kolonne einzubauen, z.B eine Kühlvorrichtung im unteren Abschnitt der Kolonne zur Auskondensation der Schwersiederfraktion und eine Kühlvorrichtung am Kopf der Kolonne zur Auskondensation der Leichtsiederfraktion. Die Fraktion mit der Acrylsäure bzw. Methacrylsäure wird im mittleren Teil der Kolonne über einen oder mehrere Seitenabzüge abgezogen. Es wird somit bei der Kondensation die Lösung, die in Stufe (b) kristallisiert wird, als Mittelsiederfraktion abgezogen. Der in der Kolonne vorliegende Druck hängt von der Menge an nicht kondensierbaren Komponenten ab und beträgt vorzugweise 0,5 - 5 bar Absolutdruck, insbesondere 0,8 - 3 bar Absolutdruck. Die genauen Betriebsbedingungen für die Kolonne, wie Temperatur- und Druckführung, Schaltung und Anordnung der Kühlvorrichtung(en), Anordnung des Seitenabzugs/der Seitenabzüge zum Abziehen der Acryl- bzw. Methacrylsäure, Wahl der Kolonnenhöhe und des Kolonnendurchmessers, Anzahl und Abstand der trennwirksamen Einbauten/Böden in der Kolonne oder Art der trennwirksamen Kolonneneinbauten, können vom Fachmann im Rahmen fachüblicher Versuche in Abhängigkeit von der Trennaufgabe ermittelt werden. In einer bevorzugten Ausführungsform wird das heiße Gasgemisch vor der Auskondensation direkt oder indirekt abgekühlt. Zu bevorzugen ist im Fall der direkten Kühlung, daß das Gasgemisch mit Hilfe der aus dem Gasgemisch kondensierten Schwersiederfraktion abgekühlt wird. Im anderen Fall wird ein Hilfsstoff in das Verfahren eingetragen, der jedoch wieder aufgearbeitet werden muß. Apparativ kann diese Vorkühlung im Sumpfbereich der Kolonne integriert (mit oder ohne Kolonneneinbauten) oder getrennt von der Kolonne in einem eigenen Apparat, z.B. einem Gaskühler, einem Quench oder einem Flashtopf erfolgen. In einer besonders bevorzugten Ausgestaltung der Erfindung läuft die Kondensation des gasförmigen Reaktionsgemisches in einer Kolonne wie folgt ab, wobei sich die Kolonne in verschiedene Abschnitte gliedern läßt, in denen die folgenden unterschiedlichen verfahrenstechnischen Aufgaben gelöst werden:
- Sumpfbereich:
   Abkühlung des heißen Gasgemisches
   Im Sumpfbereich wird das heiße Gasgemisch eingeleitet und abgekühlt. Dies kann über indirekte Kühlung, z.B. Wärmetauscher, oder direkte Kühlung mit im nächsten Abschnitt der Kolonne kondensierter Schwersiederfraktion als Kühlmedium erfolgen.
- Erster Kühlkreis:
   Kondensation der Schwersiederfraktion
   Im Bereich des ersten Kühlkreises wird die Kondensationswärme extern über den ersten Kühlkreis mittels eines Wärmetauschers mit z. B. Wasser als Kühlmedium abgeführt, indem kondensierte Schwersiederfraktion aus der Kolonne abgeführt wird, mittels des Wärmetauschers gekühlt wird und ein Teil der gekühlten, kondensierten Schwersiederfraktion der Kolonne rückgeführt wird, während der andere Teil, üblicherweise weniger als 1 Gew.-% bezogen auf 100 Gew.-% Kondensat im Seitenabzug, ausgeschleust wird. Die rückgeführte, kondensierte Schwersiederfraktion wird im Gegenstrom zum aufsteigenden Gas geführt.
- Erster Kühlkreis bis Seitenabzug:
   Schwersiederanreicherung
   Zwischen erstem Kühlkreis und dem Seitenabzug erfolgt zum ersten Kühlkreis hin eine destillative Anreicherung und Auskondensation der Schwersiederfraktion aus dem im Gegenstrom nach oben geführten Gasstrom.
- Seitenabzug:
   Abziehen der Säure
   Über den Seitenabzug wird die Acrylsäure bzw. Methacrylsäure abgeführt.
- Seitenabzug bis zweiter Kühlkreis:
   Anreicherung der Mittelsiederfraktion
   Im Bereich zwischen dem Seitenabzug und dem zweiten Kühlkreis erfolgt die Anreicherung der Mittelsiederfraktion aus dem im Gegenstrom nach oben geführten Gasstrom, wobei die Mittelsiederfraktion zum Seitenabzug hin angereichert wird.
- Zweiter Kühlkreis:
   Kondensation der Leichtsiederfraktion

Im Bereich des zweiten Kühlkreises erfolgt die Kondensation der Leichtsiederfraktion aus dem im Gegenstrom nach oben geführten Gasstrom. Die Kondensationswärme wird extern über den zweiten Kühlkreis mittels eines Wärmetauschers mit z. B. Wasser als Kühlmedium abgeführt, indem kondensierte Leichtsiederfraktion abgezogen, gekühlt und ein Teil der gekühlten, kondensierten Leichtsiederfraktion der Kolonne rückgeführt wird, während der andere Teil ausgeschleust wird. Die nicht kondensierten Komponenten, bei denen es sich vorzugsweise um Stickstoff, Kohlenmonoxid, Kohlendioxid, Sauerstoff, Methan, Propan und Propen handelt, werden vom Kopf der Kolonne abgezogen.

Daneben kann die Kondensation nach üblichen Verfahren ein- oder mehrstufig erfolgen, wobei die Art der Kondensation keiner besonderen Beschränkung unterliegt. Vorteilhafterweise wird die Kondensation mit einem Direktkondensator durchgeführt, wobei bereits erzeugtes Kondensat mit dem heißen gasförmigen Reaktionsprodukt in Kontakt gebracht wird. Als Apparate für die Kondensation eignen sich insbesondere Sprühwäscher, Venturiwäscher, Blasensäulen oder Apparate mit berieselten Oberflächen.

Die durch Partial- oder Totalkondensation des Reaktionsprodukts aus dem hergestellten gasförmigen Produktgemisch erhaltene Mischung, insbesondere das Kondensat der Mittelsiederfraktion bei Kondensation in einer Kolonne, enthält vorzugsweise 60 bis 99,5 Gew.-% Acrylsäure bzw. Methacrylsäure, 0,1 bis 40 Gew.-% Wasser, daneben 0,1 bis 15 Gew.-% Verunreinigungen, insbesondere, jeweils bezogen auf 100 Gew.-% Kondensat, 0,01 bis 5 Gew.-% (Meth)acrolein, 0,05 bis 5 Gew.-% Essigsäure, 0,01 bis 5 Gew.-% Propionsäure, 0,01 bis 5 Gew.-% Formaldehyd, 0,01 bis 5 Gew.-% weitere Aldehyde und 0,01 bis 5 Gew.-% Maleinsäure. Besonders bevorzugt wird bei der Kondensation ein Gemisch erhalten, welches 93 bis 98 Gew.- % Acrylsäure bzw. Methacrylsäure, 1 bis 5 Gew.-% Wasser, daneben 0,5 bis 5 Gew.-% Verunreinigungen, insbesondere, jeweils bezogen auf 100 Gew.-% Kondensat, 0,01 bis 3 Gew.-% Acrolein bzw. Methacrolein, 0,1 bis 3 Gew.-% Essigsäure, 0,01 bis 3 Gew.-% Propionsäure, 0,01 bis 3 Gew.-% Formaldehyd, 0,01 bis 3 Gew.-% weitere Aldehyde und 0,01 bis 3 Gew.-% Maleinsäure enthält.

### Stufe (b):

In Stufe (b) wird die in Stufe (a) erhaltene Lösung, die Acrylsäure bzw. Methacrylsäure enthält, kristallisiert. Somit wird die in der Kondensationsstufe erhaltene Lösung direkt der Kristallisation zugeführt. Hierbei wird ohne Zusatz eines Lösungsmittels, insbesondere ohne Zusatz eines organischen Lösungsmittels, gearbeitet. Das verwendete Kristallisationsverfahren unterliegt keiner Beschränkung. Die Kristallisation kann kontinuierlich oder diskontinuierlich, einstufig oder mehrstufig durchgeführt werden. Vorzugsweise erfolgt die Kristallisation einstufig. In einer anderen bevorzugten Ausführungsform der Erfindung wird die Kristallisation als fraktionierte Kristallisation durchgeführt. Üblicherweise werden bei fraktionierter Kristallisation alle Stufen, die ein Kristallisat erzeugen, das reiner ist als die zugeführte wäßrige Acrylsäurelösung bzw. Methacrylsäurelösung, Reinigungsstufen genannt und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Vorteilhafterweise liegt die Temperatur der Lösung während der Kristallisation zwischen +5 °C und +14 °C, insbesondere zwischen 8 °C und 12 °C. Der Feststoffgehalt im Kristallisator liegt vorteilhafterweise zwischen 0 und 80 g/100 g, bevorzugt zwischen 15 und 35 g Feststoff/100 g.

In einer vorteilhaften Ausgestaltung der Erfindung erfolgt die Kristallisation durch Kühlung von Apparatewänden oder durch Verdampfung der Lösung im Vakuum. Bei der Kristallisation durch Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere bevorzugte Ausführungsform bei der Kühlungskristallisation ist die Verwendung von Kühlscheibenkristallen, wie sie z.B. von der Fa. Gouda (Holland) hergestellt werden. Bei einer weiteren geeigneten Variante zur Kristallisation durch Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen. In einer weiteren vorteilhaften Ausführung der Erfindung erfolgt die Kristallisation in Apparaten, in denen die Kristalle im Kristallisationsapparat an gekühlten Flächen aufwachsen, d.h. im Apparat fixiert sind (z.B. Schichtkristallisationsverfahren der Fa. Sulzer Chemtech (Schweiz) oder Statisches Kristallisationsverfahren der Fa. BEFS PROKEM (Frankreich)).

### Stufe (c):

In Stufe (c) werden die in Stufe (b) erhaltenen Acrylsäurekristalle bzw. Methacrylsäurekristalle von der Mutterlauge abgetrennt. Für den Fall der Schichtkristallisation oder der Statischen Kristallisation kann die Trennung der Kristalle von der Mutterlauge im Kristallisationsapparat selbst erfolgen, da die Kristalle im Apparat fixiert sind und die Mutterlauge durch Abließenlassen aus dem Apparat entfernt werden kann. Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. In einer bevorzugten Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Beim Waschen liegt die Waschflüssigkeitsmenge geeigneterweise zwischen 0 und 500 g Waschflüssigkeit/100 g Kristallisat, vorzugsweise zwischen 30 und 200 g Waschflüssigkeit/100 g Kristallisat. Die verwendete Waschflüssigkeit unterliegt keiner Einschränkung. Vorteilhafterweise wird jedoch mit Reinprodukt gewaschen, d.h. mit einer Flüssigkeit, die Acrylsäure bzw. Methacrylsäure enthält, deren Reinheit höher ist als die des zu waschenden Kristallkuchens. Daneben ist auch eine Wäsche mit Wasser möglich. Das Waschen kann in hierfür üblichen Apparaten erfolgen. Vorteilhafterweise werden Waschkolonnen, in denen die Abtrennung der Mutterlauge und das Waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche. Vorteilhafterweise beträgt die Schwitzmenge zwischen 0 und 100 g abgeschmolzenes Kristallisat/100 g Kristallisat vor dem Schwitzen, vorzugsweise zwischen 5 und 35 g abgeschmolzenes Kristallisat/100 g Kristallisat. Besonders bevorzugt ist die Durchführung des Schwitzens auf Zentrifugen oder Bandfiltern. Auch die Durchführung einer Kombination aus Waschen und Schwitzen in einem Apparat kann geeignet sein.

Die Acrylsäurekristalle bzw. Methacrylsäurekristalle nach der Fest-Flüssig-Trennung und ggf. weiterem Waschen und/oder Schwitzen stellen die gereinigte Säure aus dem Verfahren dar. Die Reinheit der erhaltenen Kristalle beträgt in der Regel 97 bis 99,99 Gew.-% Acrylsäure bzw. Methacrylsäure, insbesondere 98,5 bis 99,9 Gew.-% Acrylsäure bzw. Methacrylsäure. Die nach dem erfindungsgemäßen Verfahren hergestellten Kristalle enthalten nurmehr ganz geringe Mengen an Verunreinigungen, wie Essigsäure, Maleinsäure oder Aldehyde.

Falls gewünscht, kann die gereinigte Säure nach bekannten Methoden verestert oder nach bekannten Methoden weiter gereinigt werden.

### Stufe (d):

In Stufe (d) wird die nach Abtrennung der Kristalle zurückbleibende Mutterlauge aus Stufe (c) wenigstens teilweise direkt in die Kondensationsstufe (a) zurückgeführt. Der Anteil der zurückgeführten Mutterlauge liegt zwischen 80 und 100 Gew.-%, vorzugsweise beträgt er 100 Gew.-%.

Die Figur zeigt ein bevorzugtes Ausführungsbeispiel zur Durchführung des erfindungsgemäßen Verfahrens. Über Leitung 2 und Verdichter 3 wird den Synthesereaktoren 4 und 5 Luft zugeführt. Zusätzlich wird dem Reaktor 4 über die Leitung 9 von dem Verdichter 6 verdichtetes Kreisgas, das im wesentlichen aus Stickstoff, Kohlenoxiden und nicht umgesetzten Edukten besteht, zusammen mit aus der Leitung 1 stammendem Propen bzw. Isobuten zugeführt. Im Synthesereaktor 4 findet die erste Stufe der zweistufigen Gasphasenoxidation statt, nämlich die Oxidation von Propen bzw. Isobuten zu dem entsprechenden Acrolein. Im Synthesereaktor 5 wird dann das Acrolein zu der entsprechenden Säure oxidiert. Hierbei entsteht ein gasförmiges Produktgemisch, das neben der Säure weitere, oben genannte Verunreinigungen enthält. Dieses wird über die Leitung 7 dem Kondensator 8 zugeführt, in dem es abgekühlt und kondensiert wird. Der Kondensator 8 ist in der Figur als Kolonne ausgebildet. Der nichtkondensierte Teil des Produktgemischs wird über die Leitung 9 abgeführt, von der ein Teil als Kreisgas, wie oben beschrieben, dem Reaktor 4 zurückgeführt wird und der andere Teil, vorzugsweise 50% des Gesamtstroms der Leitung 9, als Abgas aus der Anlage über die Leitung 10 abgeführt wird. Die kondensierte Schwersiederfraktion wird über die Leitung 18 abgeführt, während die kondensierte Leichtsiederfraktion über Leitung 19 abgeführt wird. Die kondensierte Mittelsiederfraktion, die den größten Teil der Acrylsäure bzw. Methacrylsäure enthält, wird über die Leitung 11 (Seitenabzug) der Kristallisationsvorrichtung 12 zugeführt, in der die Kristallisation durchgeführt wird. Die Mutterlauge aus der Kristallisation wird zusammen mit dem Kristallisat über Leitung 13 einem geeigneten Apparat 14 zur Fest-Flüssig-Trennung zugeführt, wobei über die Leitung 15 das Kristallisat und über die Leitung 16 die Mutterlauge abgeführt wird. Wenigstens ein Teil der Mutterlauge wird über die Leitung 17 in den Kondensator 8 geführt, vorzugsweise im Bereich des Seitenabzugs (Leitung 11), und somit der Kondensation wieder zugeführt. Somit wird über die Leitung 15 die gereinigte Rohsäure abgeführt.

Durch die Rückführung der Mutterlauge in die Kondensationsstufe ermöglicht die vorliegende Erfindung eine hohe Ausbeute bis zu 99,5 %. Das erfindungsgemäße Verfahren eignet sich insbesondere zur Abtrennung von Acrylsäure bzw. Methacrylsäure aus solchen Reaktionsgasgemischen, die signifikante Mengen an Wasserdampf enthalten.

Das erfindungsgemäße Verfahren bietet gegenüber den bisher bekannten Verfahren weiterhin den Vorteil, daß nach Kondensation des bei der Gasphasenoxidation entstehenden Produktgemischs direkt aus der bei der Kondensation entstehenden Lösung durch Kristallisation eine Rohsäure mit sehr guter Qualität erhalten wird. Beim Einsatz einer Kristallisation mit mehr als einer Reinigungsstufe kann direkt eine Reinsäure erzeugt werden, wobei anders als in den oben genannten Schriften, kanadisches Patent 790 625, JP-A-0 07 082 210-A und EP-A-0 616 998 keine Vorreinigung erfolgen muß.

Ein weiterer wichtiger Vorteil des erfindungsgemäßen Verfahrens ist, daß das Verfahren relativ kalt durchgeführt wird, d.h. der Hauptstrom an Acrylsäure wird direkt über Kondensation und Kristallisation als Produkt aus dem Prozeß geführt. Da anders als im Stand der Technik kein Hilfsstoff zugesetzt wird und somit keine hohe thermische Belastung (insbesondere bei hohen Acrylsäuregehalten) zur Abtrennung dieses Hilfsstoffes erforderlich ist, werden Polymerisationsprobleme und der Einsatz von Prozeßstabilisatoren, wie sie im Stand der Technik hierbei auftreten, verringert. Außerdem wird damit auch das Fouling vermieden oder reduziert. Es ist überraschend, daß es möglich ist, durch Gasphasenoxidation und Kondensation erhaltene Acrylsäurelösungen oder Methacrylsäurelösungen direkt kristallisieren zu können, und daß hiermit Produkte sehr hoher Reinheit erhalten werden. Insbesondere ist es überraschend, daß dies auch bei wäßrigen Kondensaten möglich ist.

Die Erfindung wird anhand des folgenden Beispiels, das eine bevorzugte Ausführungsform der Erfindung darstellt, näher erläutert.

### Beispiel

Es wurde folgendes gasförmige Produktgemisch mit einer Temperatur von 270 °C durch katalytische Gasphasenoxidation von Propen erhalten.

**Tabelle 1**

| **Komponente** | **Konzentration Gew.-%** |
|---|---|
| Wasser | 4,358 |
| Formaldehyd | 0,20 |
| Essigsäure | 0,43 |
| Acrylsäure | 10,1 |
| Maleinsäureanhydrid | 0,07 |
| Benzoesäure | 0,02 |
| Acrolein | 0, 1 |
| Phthalsäureanhydrid | 0,01 |
| Propionsäure | 0,002 |
| Maleinsäure | 0 |
| Allylacrylat | 0,001 |
| Benzaldehyd | 0,001 |
| Furfural | 0,002 |
| Phenothiazin | 0 |
| Stickstoff | 76,4 |
| Sauerstoff | 3,6 |
| Kohlenoxid | 0,75 |
| Kohlendioxid | 2,62 |
| Propen | 0,52 |
| Propan | 0,73 |

Das Gemisch (10931 g/h) wurde der Kondensationsstufe (a) zugeführt. Als Kondensationsapparat wurde eine Bodenkolonne mit 27 Glockenböden verwendet. Die Temperatur im Kolonnensumpf betrug 100 °C. Die Kondensationswärme wurde über Wärmeüberträger an den Böden 1 und 27 abgeführt. Am Kopf der Kolonne wurde Phenothiazin als Stabilisator zugegeben. Am Boden 27 wurde ein Strom von 425 g/h folgender Zusammensetzung abgezogen:

**Tabelle 2**

| **Komponente** | **Konzentration Gew.-%** |
|---|---|
| Wasser | 89,47 |
| Formaldehyd | 0,125 |
| Essigsäure | 6,345 |
| Acrylsäure | 4,0 |
| Maleinsäureanhydrid | < 0,0001 |
| Benzoesäure | < 0,0001 |
| Acrolein | 0,0541 |
| Phthalsäureanhydrid | < 0,0001 |
| Propionsäure | < 0,0001 |
| Maleinsäure | < 0,0001 |
| Allylacrylat | 0,0012 |
| Benzaldehyd | < 0,0001 |
| Furfural | < 0,0001 |
| Phenothiazin | < 0,0001 |
| Stickstoff | 0 |
| Sauerstoff | 0 |
| Kohlenoxid | 0 |
| Kohlendioxid | 0 |
| Propen | 0 |
| Propan | 0 |

Am Kolonnensumpf wurde ein Strom von 2 g/h folgender Zusammensetzung abgezogen:

**Tabelle 3**

| **Komponente** | **Konzentration Gew.-%** |
|---|---|
| Wasser | 1,21 |
| Formaldehyd | 0,0036 |
| Essigsäure | 0,879 |
| Acrylsäure | 39,45 |
| Maleinsäureanhydrid | 34,55 |
| Benzoesäure | 10,931 |
| Acrolein | 0,0103 |
| Phthalsäureanhydrid | 5,465 |
| Propionsäure | 0,0477 |
| Maleinsäure | < 0,0001 |
| Allylacrylat | 0,0113 |
| Benzaldehyd | < 0,2673 |
| Furfural | < 0,3639 |
| Phenothiazin | 6,8039 |
| Stickstoff | 0 |
| Sauerstoff | 0 |
| Kohlenoxid | 0 |
| Kohlendioxid | 0 |
| Propen | 0 |
| Propan | 0 |

Das Abgas hatte folgende Zusammensetzung:

**Tabelle 4**

| **Komponente** | **Konzentration Gew.-%** |
|---|---|
| Wasser | 0,982 |
| Formaldehyd | 0,239 |
| Essigsäure | 0,0305 |
| Acrylsäure | 0,0103 |
| Maleinsäureanhydrid | < 0,0001 |
| Benzoesäure | < 0,0001 |
| Acrolein | 0,1253 |
| Phthalsäureanhydrid | < 0,0001 |
| Propionsäure | < 0,0001 |
| Maleinsäure | < 0,0001 |
| Allylacrylat | < 0,0001 |
| Benzaldehyd | < 0,0001 |
| Furfural | < 0,0001 |
| Phenothiazin | < 0,0001 |
| Stickstoff | 89,054 |
| Sauerstoff | 4,1797 |
| Kohlenoxid | 0,873 |
| Kohlendioxid | 3,050 |
| Propen | 0,6054 |
| Propan | 0,850 |

Das Abgas aus der Kondensationskolonne wurde in die Reaktion zurückgeführt (60 Gew.-%) bzw. aus dem Prozeß (40 Gew.-%) ausgeschleust.

Am Boden 11 wurde ein flüssiger Strom von 4657 g/h und 84,5 °C aus der Kolonne abgezogen, der dann kristallisiert wurde. Dieser Strom hatte folgende Zusammensetzung:

**Tabelle 5**

| **Komponente** | **Konzentration Gew.-%** |
|---|---|
| Wasser | 2,52 |
| Formaldehyd | 0,0062 |
| Essigsäure | 5,899 |
| Acrylsäure | 90,972 |
| Maleinsäureanhydrid | 0,399 |
| Benzoesäure | < 0,0001 |
| Acrolein | 0,0128 |
| Phthalsäureanhydrid | < 0,0001 |
| Propionsäure | 0,0564 |
| Maleinsäure | < 0,0001 |
| Allylacrylat | 0,0548 |
| Benzaldehyd | 0,0006 |
| Furfural | 0,0492 |
| Phenothiazin | 0,0300 |
| Stickstoff | 0 |
| Sauerstoff | 0 |
| Kohlenoxid | 0 |
| Kohlendioxid | 0 |
| Propen | 0 |
| Propan | 0 |

Anschließend wurde die vom Boden 11 stammende Mischung in einem 10 1 Rührbehälter mit Wendelrührer kristallisiert. Die Kristallisationswärme wurde über den Doppelmantel des Behälters abgeführt. Die Gleichgewichtstemperatur der Lösung betrug 4,8 °C. Die bei der Kristallisation erzeugte Suspension (30 g Feststoff / 100 g Suspension) wurde auf einer Zentrifuge bei 2000 U/min (Zentrifugendurchmesser 250 mm) und einer Schleuderzeit von 1 min in Kristalle und Mutterlauge getrennt. Die Kristalle (1281 g/h) wurden anschließend mit aufgeschmolzenem Kristallisat (296 g/h) 1 min lang bei 2000 U/min gewaschen.

Die Mutterlauge (3376 g/h) wurde zusammen mit der Waschflüssigkeit in die Kondensationskolonne auf Boden 10 zurückgefahren. Die Analyse der Kristalle ergab folgende Zusammensetzung:

**Tabelle 6**

| **Komponente** | **Konzentration** |
|---|---|
| Wasser | 0,1066 |
| Formaldehyd | 0,0003 |
| Essigsäure | 0,9619 |
| Acrylsäure | 98,8816 |
| Maleinsäureanhydrid | 0,0225 |
| Benzoesäure | < 0,0001 |
| Acrolein | 0,0009 |
| Phthalsäureanhydrid | < 0,0001 |
| Propionsäure | 0,0162 |
| Maleinsäure | < 0,0001 |
| Allylacrylat | 0,0031 |
| Benzaldehyd | < 0,0001 |
| Furfural | 0,0028 |
| Phenothiazin | 0,0041 |
| Stickstoff | 0 |
| Sauerstoff | 0 |
| Kohlenoxid | 0 |
| Kohlendioxid | 0 |
| Propen | 0 |
| Propan | 0 |

Wie aus Tabelle 6 ersichtlich, ermöglicht das erfindungsgemäße Verfahren die Herstellung hochreiner Acrylsäure.

## Patentansprüche

1. Verfahren zur Abtrennung von Acrylsäure oder Methacrylsäure aus einem gasförmigen Gemisch, das als Rohprodukt der katalytischen Gasphasenoxidation von C₃-/C₄-Alkanen, -Alkenen, -Alkanolen und/oder-Alkanalen und/oder Vorstufen davon zu Acrylsäure oder Methacrylsäure vorliegt, **dadurch gekennzeichnet, dass**
a) das gasförmige Gemisch kondensiert wird,
b) Acrylsäure oder Methacrylsäure aus der in Stufe a) erhaltenen Lösung kristallisiert wird,
c) die erhaltenen Kristalle aus der Mutterlauge der Stufe b) abgetrennt werden und
d) mindestens eine Teilmenge der Mutterlauge aus Stufe c) nach der Abtrennung in die Stufe a) zurückgeführt wird.

2. Verfahren nach Anspruch 1, **dadurch gekennzeichnet**, das man in Verfahrensstufe (a) das gasförmige Gemisch fraktioniert kondensiert, wobei eine Mittelsiederfraktion abgezogen wird, die Acrylsäure oder Methacrylsäure enthält und aus der in Verfahrensstufe b) Acrylsäure oder Methacrylsäure kristallisiert wird.

3. Verfahren nach einem der Ansprüche 1 oder 2, **dadurch gekennzeichnet, dass** die Kondensation in Stufe (a) in einer Kolonne mit trennwirksamen Einbauten durchgeführt wird.

4. Verfahren nach einem der Ansprüche 1 bis 3, **dadurch gekennzeichnet, dass** die Kristallisation in Stufe (b) einstufig oder mehrstufig durchgeführt wird.

5. Verfahren nach einem der Ansprüche 1 bis 4, **dadurch gekennzeichnet, dass** die Temperatur der Lösung während der Kristallisation in Stufe (b) zwischen +5°C und + 14°C liegt.

6. Verfahren nach einem der Ansprüche 1 bis 5, **dadurch gekennzeichnet, dass** bei der Kristallisation in Stufe (b) die Wärme durch Kühlung von Apparatewänden oder durch Verdampfung der Lösung im Vakuum abgeführt wird.

7. Verfahren nach einem der Ansprüche 1 bis 6, **dadurch gekennzeichnet, dass** die Kristalle in Stufe (c) durch Filtrieren und/oder Zentrifugieren von der Mutterlauge abgetrennt werden.

8. Verfahren nach einem der Ansprüche 1 bis 7, **dadurch gekennzeichnet, dass** die in Stufe (c) abgetrennten Kristalle wenigstens einem Waschen und/oder Schwitzen unterzogen werden.

9. Verfahren nach einem der Ansprüche 1 bis 8, **dadurch gekennzeichnet, dass** in Stufe (d) zwischen 80 und 100 Gew.-%, vorzugsweise 100 Gew.-% der Mutterlauge aus Stufe (c) in die Stufe (a) zurückgeführt werden.

## Claims

1. A process for removing acrylic acid or methacrylic acid from a gaseous mixture which is the crude product of the catalytic gas phase oxidation of C₃-/C₄-alkanes, -alkenes,-alkanols and/or -alkanals and/or precursors thereof to form acrylic acid or methacrylic acid, which comprises
a) condensing said gaseous mixture,
b) crystallizing acrylic acid or methacrylic acid from the solution obtained in step a),
c) removing the resulting crystals from the mother liquor of step b), and
d) recycling at least a portion of said mother liquor from step c) into step a)

2. A process according to claim 1, wherein in step a) the gaseous mixture is subjected to a fractionating condensation, a middle boilers traction being withdrawn which includes acrylic acid or methacrylic acid, and from which acrylic acid or methacrylic acid is crystallized in step b)

3. A process as claimed in claim 1 or 2, wherein said condensing of step (a) is carried out in a column having separatory internals.

4. A process as claimed in any of claims 1 to 3, wherein said crystallizing of step (b) is effected in one or more stages.

5. A process as claimed in any of claims 1 to 6, wherein said crystallizing of step (b) is effected at a temperature of said solution within the range from +5°C to +14°C.

6. A process as claimed in any of claims 1 to 5, wherein said crystallizing of step (b) is effected by removing the heat by cooling apparatus walls or by evaporating said solution under reduced pressure.

7. A process as claimed in any of claims 1 to 6, wherein said crystals of step (c) are removed from said mother liquor by filtration and/or centrifugation.

8. A process as claimed in any of claims 1 to 7, wherein said crystals removed in step (c) are subjected to at least one washing and/or sweating step.

9. A process as claimed in any of claims 1 to 8, wherein said recycling of step (d) is effected by recycling from 80 to 100 % by weight, preferably 100 % by weight, of said mother liquor from step (c) into step (a).

## Revendications

1. Procédé e séparation d'acide acrylique ou d'acide méthacrylique à partir d'un mélange gazeux qui se présente sous la forme d'un produit bruit de l'oxydation catalytique en phase gazeuse d'alcanes, alcènes, alcanols et/ou alcanals en C3-C4 et/ou de progéniteurs de ceux-ci en acide acrylique ou méthacrylique, **caractérisé en que**
a) le mélange gazeux est condensé,
b) de l'acide acrylique ou de l'acide méthacrylique est cristallisé dans la solution obtenue dans l'étape a),
c) les cristaux obtenus sont séparés de la lessive mère de l'étape b), et
d) au moins une quantité partielle de la lessive mère de l'étape c) est recyclée dans l'étape a) après la séparation.

2. Procédé suivant la revendication 1, **caractérisé en ce que** dans l'étape (a) le mélange gazeux est soumis à une condensation fractionnée, lors de la condensation fractionnée une fraction de substances à point d'ébullition moyen est soutirée qui contient acide acrylique ou acide méthacrylique et dont l'acide acrylique ou l'acide méthacrylique est cristallisée pendant l'étape (b).

3. Procédé suivant la revendication 1 ou 2, **caractérisé en ce que** la condensation est effectuée à l'étape (a) dans une colonne comportant des éléments encastrés à effet séparateur.

4. Procédé suivant l'une des revendications 1 à 3, **caractérisé en ce que** la cristallisation dans l'étape (b) est effectuée en une étape ou plusieurs étapes.

5. Procédé suivant l'une des revendications 1 à 4, **caractérisé en ce que** la température de la solution pendant la cristallisation dans l'étape (b) est comprise entre +5°C et +14°C.

6. Procédé suivant l'une des revendications 1 à 5, **caractérisé en ce que**, lors de la cristallisation dans l'étape (b), la chaleur est évacuée par refroidissement des parois d'appareillage ou par évaporation de la solution sous vide.

7. Procédé suivant l'une des revendications 1 à 6, **caractérisé en ce que** les cristaux sont séparés de la lessive mère dans l'étape (c) par filtration et/ou centrifugation.

8. Procédé suivant l'une des revendications 1 à 7, **caractérisé en ce que** les cristaux séparés dans l'étape (c) sont soumis au moins à un lavage et/ou exsudation.

9. Procédé suivant l'une des revendications 1 à 8, **caractérisé en ce que**, dans l'étape (d), entre 80 et 100% en poids, de préférence 100% en poids, de la lessive mère de l'étape (c) sont recyclés dans l'étape (a).
